# EUROPEAN PATENT SPECIFICATION

(11) **EP 2 504 709 B1**
(45) Date of publication and mention of the grant of the patent: **05.12.2018**
(21) Application number: 10833478.0
(22) Date of filing: 20.10.2010
(51) Int. Cl.: G01N 35/10, G01N 33/53, G01N 21/07, G01N 33/543, B01L 3/00, G01N 35/00

(54) **CENTRIFUGAL MICRO-FLUIDIC DEVICE AND METHOD FOR DETECTING TARGET IN FLUID SAMPLE**
MIKROFLUIDISCHE ZENTRIFUGALVORRICHTUNG UND VERFAHREN ZUR ERKENNUNG EINES ZIELS IN EINER FLÜSSIGPROBE
DISPOSITIF MICROFLUIDIQUE CENTRIFUGE ET PROCÉDÉ DE DÉTECTION DE CIBLE DANS ÉCHANTILLON DE FLUIDE

(30) Priority: 26.11.2009 KR 20090114920
(43) Date of publication of application: 03.10.2012
(73) Proprietor: Samsung Electronics Co., Ltd., Suwon-si, Gyeonggi-do, 443-742 (KR)
(72) Inventor: KIM, In Wook, Seongnam-si Gyeonggi-do 463-927 (KR); LEE, Beom Seok, Hwaseong-si Gyeonggi-do 445-736 (KR); KIM, Kui Hyun, Hwaseong-si Gyeonggi-do 445-170 (KR); KIM, Ji Won, Suwon-si Gyeonggi-do 443-796 (KR)
(74) Representative: Grootscholten, Johannes A.M.
(86) International application number: PCT/KR2010/007212
(87) International publication number: WO 2011/065666

(56) References cited:
- EP-A2- 1 901 054
- US-A1- 2002 027 133
- US-A1- 2003 152 491
- US-A1- 2008 056 949
- US-A1- 2008 108 120
- US-A1- 2008 193 336
- US-A1- 2008 237 151
- US-A1- 2008 274 015
- US-A1- 2008 300 148
- US-A1- 2009 053 108
- US-A1- 2009 286 327

## Description

### Technical Field

The invention relates to a micro-fluidic device in accordance with the preamble of claim 1, and an immunosorbent assay method using such a micro-fluidic device in accordance with the preamble of claim 6. Such a device and method are disclosed in US 2002-027133 A1.

More particularly, the centrifugal micro-fluidic device has a plurality of micro-fluidic structures arranged in a disc type platform to simultaneously conduct several immunosorbent assays.

### Background art

Among conventional methods for immunological measurement of target materials, which are contained in a liquid sample such as blood or bodily fluid, a particle-enhanced immunoassay method has been widely used. In the method, insoluble particles fixed to an antibody or antigen are mixed with a target material contained in the sample to cause immuno-agglutination via antigen-antibody binding reaction and such agglutination extent is optically determined, wherein the antibody or the antigen is specifically combined with the target material.

However, the foregoing method requires expensive automatic analysis equipment including, for example, spectrometers, cuvettes, liquid handlers, centrifuge units, etc. and numerous manual operations.

In order to perform a rapid test, a skilled medical lab technician is needed and, although the skilled medical lab technician conducts the test, it is very difficult to attain several examinations at the same time. Especially, examinations with different processes such as biochemical analysis, immunosorbent assay, etc. require different equipment.

Disk type micro-fluidic devices which facilitate a relatively simple immunosorbent assay have been proposed. Such a micro-fluidic device is an apparatus to enable biological or chemical reaction using a small amount of fluid.

In general, a micro-fluidic structure to perform an individual function includes a chamber containing fluid, a channel through which the fluid passes, and a valve for adjusting a flow of the fluid. Such structure may be fabricated by various combinations of the foregoing components. The micro-fluidic structure is arranged on a chip type substrate to perform immunosorbent reaction and/or biochemical reaction. An apparatus called 'Lab-on-a-chip' may be fabricated to perform several processing and/or operations on a single substrate.

In order to flow or transport a fluid in a micro-fluidic structure, a driving pressure is generally required. As such a driving pressure, capillary pressure or pressure generated using an additional pump may be used. In recent years, disk type micro-fluidic apparatuses, each of which has a micro-fluidic structure placed on a disk type platform and carries out a series of operations while transporting or flowing a fluid by centrifugal force, have been proposed. Such device is sometimes referred to as a Lab CD and/or a lab-on-a-disk. Investigation into development of disk type equipment to rapidly and correctly execute desired operations in a disk type platform based on centrifugal force is still continued.

However, immunosorbent assay using a conventional disk type micro-fluidic device can detect a few analytes in a single platform since the device has a complex micro-fluidic structure formed therein. It also requires an extended time for tests because of complicated process for carrying out a series of steps.

A conventional disk type micro-fluidic apparatus with a micro-fluidic structure for immunosorbent assay is different from a conventional disk type micro-fluidic structure for biochemical analysis. Therefore, such apparatus is useful for only one of the foregoing assays, thus not attaining compatibility thereof.

### Disclosure of Invention

### Technical Problem

An exemplary embodiment provides a centrifugal micro-fluidic device with a structure suitable for simultaneously conducting multiple immunosorbent assays and an immunosorbent assay method using the same.

Another exemplary embodiment provides a centrifugal micro-fluidic device to simultaneously conduct immunosorbent assay and biochemical analysis, each having different procedures and an immunosorbent assay method using the same.

### Solution to Problem

According to the invention a micro-fluidic device according to claim 1 and a method according to claim 6 is provided. According to an aspect, there is provided a disk type micro-fluidic device including: a rotational body; a micro-fluidic structure having multiple chambers, multiple channels through which the multiple chambers are connected for fluid communication therebetween, and multiple valves arranged in the channels to control fluid flow, wherein the fluid is transported via centrifugal force generated by rotation of the rotational body; and a first binder provided in the micro-fluidic structure wherein the binder selectively binds to a target material and contains solid particles as a detection label required for expressing optical signals or, functions as the detection label by itself.

According to another aspect, there is provided a test system including; an inspection part for determining optical characteristics of separate fractions under centrifugal force generated by rotation of the rotational body, after the binder is mixed and reacts with the target material; and a control part for calculating a concentration of the target material based on the optical characteristics determined above and, in addition, the foregoing micro-fluidic device of the present invention.

According to another aspect, there is provided an assay method using a micro-fluidic device, including: preparing the micro-fluidic device having a first chamber which receives a first binder, wherein the first binder is capable of selectively binding to a target material and contains solid particles or a marker material functioning as a detection label required for expressing optical signals; loading a sample; rotating the micro-fluidic device to separate the sample into a precipitate and a supernatant; transporting the supernatant into the first chamber to enable a reaction of the supernatant with the first binder; rotating the micro-fluidic device to perform fractionation of the reactant; and detecting optical properties of the respective fraction.

According to a still further aspect, there is provided an assay method using a micro-fluidic device, including: preparing the micro-fluidic device having a first chamber which receives a first binder, wherein the first binder is capable of selectively binding to a target material and contains solid particles or a marker material functioning as a detection label required for expressing optical signals, as well as a second chamber which receives a second binder, wherein the second binder is capable of selectively binding to a different site of the same target material; loading a fluid sample; rotating the micro-fluidic device to separate the sample into a precipitate and a supernatant; transporting the supernatant into the first chamber to enable a reaction of the supernatant with the first binder; transporting the reactant into the second chamber to enable another reaction of the reactant with the second binder; rotating the micro-fluidic device to perform fractionation of the reactant; and detecting optical properties of the fraction.

As such, the disk type micro-fluidic device according to the aspect may simultaneously conduct several immune reactions and also proceed such immune reactions together with biochemical reactions.

### Brief Description of Drawings

These and/or other aspects will become apparent and more readily appreciated from the following description of the embodiments, taken in conjunction with the accompanying drawings of which:
FIG. 1 is a schematic view illustrating an appearance of a disk type micro-fluidic device according to an exemplary embodiment;
FIG. 2 is a plan view illustrating a disk type micro-fluidic device equipped with an immunosorbent assay unit and a biochemical analysis unit according to an exemplary embodiment;
FIG. 3 is a detailed view illustrating an example of each of a sample chamber and a sample dispenser mounted on a disk type micro-fluidic device according to an exemplary embodiment;
FIG. 4 is a cross-sectional view illustrating an example of a valve in a closed state;
FIG. 5 is cross-sectional view illustrating an opening process of the valve shown in FIG. 4;
FIG. 6 is a block diagram illustrating a test system using a micro-fluidic device according to an exemplary embodiment; and

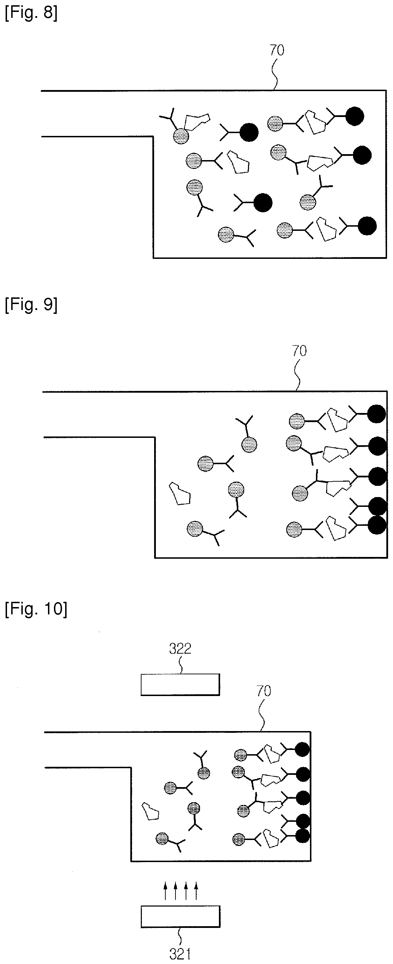

FIGS. 7 to 10 are schematic views illustrating a process of conducting immunosorbent assay using a micro-fluidic device according to an exemplary embodiment.

### Best Mode for Carrying out the Invention

Hereinafter, advantageous features and characteristics of embodiments and practical methods thereof will be clearly understood by the following detailed description with reference to the accompanying drawings. In this regard, configuration components such as a chamber or channel shown in the drawings are simply illustrated and may be scaled up or down, thus not corresponding to real sizes thereof. In terms 'micro-fluidic device,' 'micro-particle,' etc., the prefix "micro-" was used herein only in contrast with "macro-" and does not limitedly refer to a unit of size.

'Micro-fluidic structure' used herein means a structure including various microstructural elements such as plural chambers, channels and/or valves in order to flow a fluid, rather than a specific form of structure. Therefore, 'micro-fluidic structure' may form a unit device to attain different functions based on features in arrangement of chambers, channels and valves and types or kinds of materials contained therein.

'Reactive solution' as used herein refers to all solutions capable of producing a final product that can be detected by various means depending on a detectable label used. However, in order to distinguish a reactive solution used for immunosorbent assay from another reactive solution used for biochemical analysis, the latter is expressed as "biochemical reactive solution."

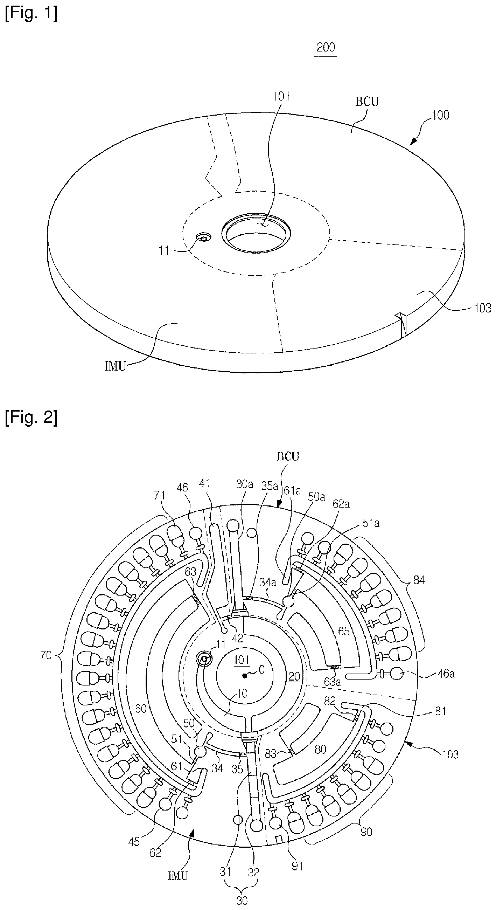

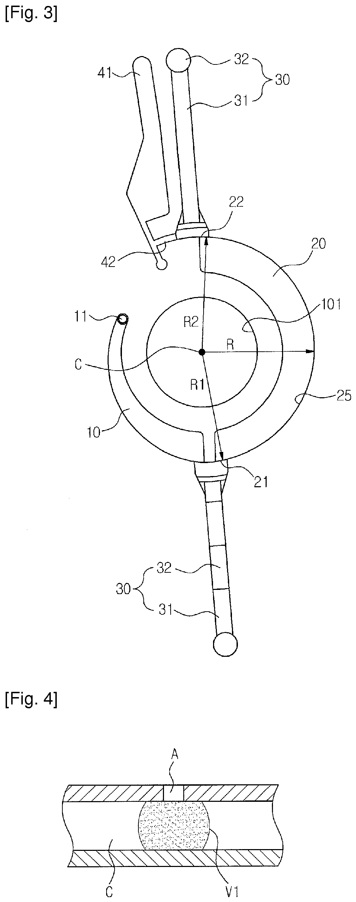

FIG. 1 shows a schematic appearance of a disk type micro-fluidic device according to an exemplary embodiment of the present invention; FIG. 2 is a plan view illustrating a disk type micro-fluidic device equipped with an immunosorbent assay unit (IMU) and a biochemical analysis unit (BCU) according to an exemplary embodiment of the present invention; FIG. 3 is a detailed view illustrating an example of each of a sample chamber and a sample dispenser mounted on a disk type micro-fluidic device according to an exemplary embodiment of the present invention.

The exemplary embodiment shown in FIG. 2 is a disk type micro-fluidic device 200 including at least one immunoassay unit IMU and at least one biochemical analysis unit BCU. However, the micro-fluidic device may have only one or more immunosorbent assay units in a rotational disk type platform 100.

Shape of the disk type platform 100 is not restricted to a circular shape but may include a sector form placed on a rotatable frame, other than a rotational circular disk shape. A disk type platform 100 is easily formed and a surface of the disk type platform may be fabricated using at least one selected from a variety of plastic materials with biological inactivity, such as polymethylmethacrylate (PMMA), PDMS, PC, etc. However, the material for the disk type platform is not particularly restricted and may include any materials with chemical an/or biological stability, favorably optical transparency and mechanical workability.

Such platform 100 may be fabricated in a laminate form of multiple plates. By forming depressions on each facing surface of two plates wherein the depressions correspond to a chamber or channel, and then, binding both plates, an empty space and/or a passage is provided inside the platform 100.

Binding of plates may be performed by any conventional methods such as application of an adhesive or a double-sided adhesive tape, ultrasonic welding, etc.

IMU is a unit for detection of a target material, for example, a specific antibody, antigen or protein by receiving blood or a serum and processing the blood or serum in a micro-fluidic structure (not shown) mounted on a part of the disk type plat form 100. The IMU may include a plurality of chambers, channels for connecting at least two chambers, and a micro-fluidic structure (not shown) having at least one valve for controlling a flow of fluid.

BCU receives a biological sample as blood, serum, urine, saliva, etc., and the target material is detected by a biochemical reaction of the sample with a pre-stored reactive solution. The reactive solution chemically reacts with the sample to enable optical detection of the target material.

Such micro-fluidic device 200 may be fixed to a rotational driver 301 for high speed rotation. At the center of the micro-fluidic device 200, a built-in thru-hole 101 is formed to mount the micro-fluidic device on the rotational driver 301. Centrifugal force generated by rotation of the rotational driver 301 serves to transfer and/or blend the sample.

Using the disk type micro-fluidic device 200 according to an aspect of the present invention may perform immunoassay such as troponin I (TnI) as a cardiac marker, beta-hCG indicating pregnancy, a number of airborne allergens, specific human immunoglobulin E, etc. and/or simultaneously conduct biochemical analysis including, for example, liver test panels such as ALT (Alanine Aminotransferase: GPT) or AST (Aspartate Aminotransferase: GOT), digestive test such as amylase and/or lipase for determination of abnormal digestive system (especially, pancreas), and so forth.

However, the present invention is not particularly limited to the foregoing analyses and, if necessary, other test items may be added or the above analysis items may be replaced with others.

### Table 1

### [Table 1]

**[Table]**

| Test field | Immunosorbent assay | Biochemical analysis | Note |
|---|---|---|---|
| Emergency chemical test | Cardiac marker (CK-MB, TnI, myoglobin, pro-BNP), β-hCG | Liver test (ALT, AST), glucose, digestive test (amylase, lipase) | Emergency room |
| Hepatitis and liver function test | HBsAg, Anti-HBs, Anti-HBc, Anti-HCV | Liver panel (AST, ALT, TB, Albumin, GGT) | General (ALT: periodic monitoring item of chronic B hepatitis patients) |
| Blood sugar test | HbA1C | Glucose | General (HbA1C: average blood sugar level over three months) |
| Cardiovascular disease test | Cardiac marker | Liver panel (TC, HDL, LDL, TG) | Cardiology |
| Thyroid test | Free T4, TSH | Glucose | Endocrinology |

| | | | |
|---|---|---|---|
| CK-MB: Creatine Kinase-MB (CK-MB) TnI: Cardiac-specific Troponin I AST: Aspartate transaminase ALT: Alanine transaminase GGT: Gamma-glutamyl transferase TB: Tuberculin Skin Test HbsAg: hepatitis B surface antigen HbAlc: Glycated hemoglobin TSH: Thyroid-stimulating hormone HDL: High-density lipoprotein LDL: Low -density lipoprotein TG: Thyroglobulin | | | |

In TABLE 1, some examples of combination of immunosorbent assay and biochemical analysis capable of providing significant information of a certain ailment by concurrently performing the same are listed. Other than the listed tests, a variety of desired test items may also be combined.

A sample chamber 10 is placed at a position close to the rotation center ("C" in FIG. 2) of the platform 100. A sample is received in the sample chamber 10. The sample chamber 10 may have an inlet 11 to load the sample into the chamber.

The sample dispenser 30 accepts the sample from the sample chamber 10 and, for example, has a certain volume to measure a constant amount of sample for testing. In order to transport the sample from the sample chamber 10 to the sample dispenser 30, centrifugal force generated by rotation of a platform 100 is applied. Therefore, the sample dispenser 30 is positioned closer to the perimeter than the sample chamber 10. The sample dispenser 30 may serve as a centrifuge to separate the sample, for example, blood into a supernatant and a precipitate by rotation of the platform 100. The sample dispenser 30 for centrifugation may have different forms and an illustrative example thereof is shown in FIGS. 2 and 3. The sample dispenser 30 may have a supernatant collector 31 in a channel form outwardly extending in a radial direction and a precipitate collector 32 placed at an end of the supernatant collector 31, providing a space to collect the precipitate with relatively greater specific gravity.

The sample dispenser 30 of the IMU is directly connected to the sample chamber 10 to receive the sample. A sample dispenser 30a of the BCU is connected to the sample dispenser 30 via a sample transport part 20, by which the sample is transported from the sample chamber 10 to the sample dispenser 30 to fill the sample dispenser 30 and in turn fill the sample dispenser 30a via the sample transport part 20.

Referring to FIG. 3, the sample transport part 20 has a first connection part 21 following the sample dispenser 30 and a second connection part 22 following the sample dispenser 30a. The first and second connection parts 21 and 22 may be connected to the outer wall 25 of the sample transport part 20. A distance R2 from the center of rotation C to the second connection part 22 in a radial direction may be larger than a distance R1 from the center of rotation C to the first connection part 21. A radius of curvature R of the outer wall 25 between the first and second connection parts 21 and 22 may be larger than R1 and gradually increased from the first connection part 21 toward the second connection part 22. According to such configuration, centrifugal force generated by the rotation of the micro-fluidic device may move the sample toward the sample dispenser 30 and fill the first sample dispenser 31 and in turn move again toward the sample transport part 20. Following this, the sample flows along the outer wall 25 of the sample transport part 20 by the centrifugal force and is delivered to the second sample dispenser 32 through the second connection part 22. If a plurality of sample dispensers is used to receive the sample from the sample chamber, inconvenience of injecting the sample into separate units for immunoassay and biochemical analysis may be eliminated.

As shown in FIG. 2, a sample dispensing channel 34 is positioned at a side of the supernatant collector 31 to dispense the collected supernatant (i.e., serum when blood is used as a sample) to a next structure. The sample dispensing channel 34 is connected to the supernatant collector 31 via a valve 35.

The valve 35 may be a micro-fluidic valve with different morphologies. For example, a capillary valve passively opened by a pre-determined pressure or an alternative valve with active behavior by external power or energy (i.e., magnetic energy or heat energy) may be adopted. In one exemplary embodiment, the valve 35 may change between solid and fluid phase, and is a normally closed valve to close the channel 34 in order to prevent a fluid from flowing until electromagnetic energy is applied to change it from solid to fluid phase to open the channel.

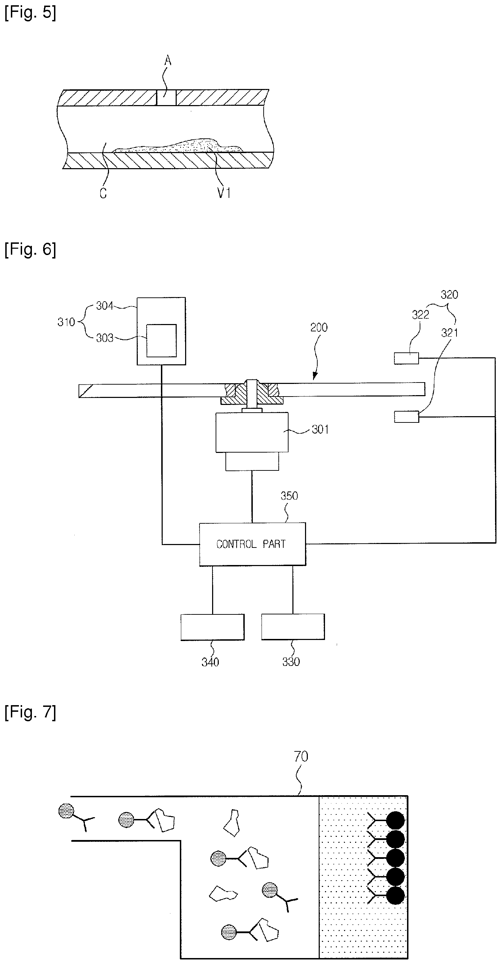

FIG. 4 is a cross-sectional view illustrating an example of the closed valve while FIG. 5 is another cross-sectional view illustrating a process of opening the valve shown in FIG. 4.

The closed valve may contain a valve material V1 in a solid state at room temperature. The valve material V1 is present in a solid condition in the channel C so as to block the channel C, as shown in FIG. 4. Such valve material V1 melts at a high temperature to flow into a space of the channel C and, as shown in FIG. 5, opens the channel C. An example of external energy is electromagnetic energy and an energy source may include, for example, a laser source radiating a laser beam, a light emitting diode radiating visible or infrared light, a xenon lamp, and the like. The laser source may have at least one laser diode. Such external energy source may be suitably selected in consideration of wavelength of electromagnetic energy absorbed by thermal particles contained in the valve material V1. The valve material V1 may be a thermoplastic resin including, for example: cyclic olefin copolymer (COC); polymethylmethacrylate (PMMA); polycarbonate (PC); polystyrene (PS); polyoxymethylene (POM); perfluoralkoxy (PFA); polyvinylchloride (PVC); polypropylene (PP); polyethylene terephthalate (PET); polyether ether ketone (PEEK); polyamide (PA); polysulfone (PSU); polyvinylidene fluoride, etc. Alternatively, the valve material V1 may be a phase transition substance in a solid state at room temperature. Such phase transition substance may comprise wax. When the wax is heated, it melts into a liquid and volume thereof expands. The wax may include, for example, paraffin wax, microcrystalline wax, synthetic wax, natural wax, etc. The phase transition substance may be a gel or a thermoplastic resin. The gel may include, for example, polyacrylamide, polyacrylate, polymethacrylate, polyvinylamide, etc. The valve material V1 may contain numerous micro-thermal particles dispersed therein, wherein the particles absorb electromagnetic energy and generate heat. Each of such micro-thermal particles has a diameter of 1nm to 10µm to freely pass through the channel with about 0.1mm depth and 1mm width. The micro-thermal particles generate heat and are uniformly dispersed throughout the wax, for example, when electromagnetic energy is incident from a laser and in turn rapidly elevates a temperature. In order to embody such features, the micro-thermal particle may have a core containing metal ingredients and a hydrophobic surface structure. For instance, the micro-thermal particle may have a specific molecular structure with an Fe-based core and a plurality of surfactants bonded to Fe to enclose the same. The micro-thermal particles may be dispersed in a carrier oil for storage. In order to homogeneously disperse the micro-thermal particles having a hydrophobic surface structure in the carrier oil, the carrier oil may be hydrophobic. After pouring the carrier oil containing the micro-thermal particles dispersed therein into the molten phase transition substance and mixing the same, the mixture is introduced into the channel C and in turn is solidified therein, thus blocking the channel C. The micro-thermal particle is not restricted to a polymer particle illustrated above and may adopt a quantum dot or magnetic bead form. The micro-thermal particle may comprise, for example, micro-metal oxides such as Al₂O₃, TiO₂, Ta₂O₃, Fe₂O₃, Fe₃O₄, HfO₂ and the like. The closed valve does not have to contain the micro-thermal particles and, instead, may consist of a phase transition substance without micro-thermal particles. In this case, a non-contact type heater may be provided a desired distance apart from the micro-fluidic device to heat and melt the valve substance, so as to heat a target valve to be open.

Referring to FIG. 2, the channel 34 is connected to a supernatant metering chamber 50 in which the supernatant isolated from the sample is received. The supernatant metering chamber 50 is connected to a dilution chamber 60 via a valve 51. As the valve 51, a micro-fluidic valve with the same morphology as the valve 35 described above may be used.

The dilution chamber 60 may contain a desired amount of dilution buffer in consideration of dilution ratio of the supernatant required for assay.

The supernatant inflow in the dilution chamber 60 is diluted by the dilution buffer present in the dilution chamber 60.

The supernatant metering chamber 50 may be designed to have a volume capable of receiving a pre-determined amount of sample in consideration of dilution ratio. So far as the valve 51 is in closed state, an excess amount of the sample larger than the volume of the supernatant metering chamber 50 cannot be introduced into the same chamber 50. As a result, the pre-determined amount of the supernatant may be fed into the dilution chamber 60.

At least two reaction chambers 70 are arranged parallel to be radially outwardly connected to and receive a diluted sample from the dilution chamber 60. The reaction chambers 70 are connected to the dilution chamber 60 through a dispenser channel 61. Distribution of a diluted sample through the dispenser channel 61 may be controlled by a valve 62. An alternative valve 63 functions to form an air vent path to enable simple distribution of the diluted sample into the reaction chambers 70. Such valves 62 and 63 may be micro-fluidic valves with the same morphologies as the value 35.

The reaction chambers 70 receives a first binder composite as a reagent in a liquid or dried solid state.

The first binder is coupled to particles or fluorescent dye with a relatively small specific gravity, compared to particles of a second binder described below, for example, gold nanoparticles or polystyrene nanoparticles, quantum dots, and the like.

The reagent containing the first binder may be preloaded into the micro-fluidic device. For example, it may be loaded before binding an upper plate with a lower plate to form a platform 100 in a process for fabrication of a micro-fluidic device. Alternatively, the reaction chamber 70 may be a chamber having a vent and/or an inlet instead of a closed reaction chamber and, in this case, the reagent may be introduced into the reaction chamber 70 before being subjected to assay.

For instance, before both upper and lower plates are combined to form a platform 100 during manufacture of a micro-fluidic device, a liquid reagent containing a first binder is introduced into at least two reaction chambers 70 and those contained in the reaction chambers are simultaneously subjected to lyophilization according to a known lyophilizing program. Afterward, binding the upper and lower plates together, the micro-fluidic device 200 containing the lyophilized reagent therein may be fabricated.

After diluting a sample in a dilution chamber 60 to a desired concentration, a valve 62 is opened and the sample flows toward the reaction chambers 70 and in turn dissolves the dried first binder, thus forming a first composite.

In the reaction chambers 70, the first binder and a target substance in the sample are brought in contact with each other for a desired period of time to allow to form a first composite (or complex) of the first binder and the target substance, for example through an immune reaction.

Next, after the formation of the first composite, the micro-fluidic device 200 rotates to enable centrifugation of the composite.

According to rotation of the micro-fluidic device 200, the first composite moves outwardly in a radial direction of the reaction chamber 70 owing to centrifugal force while unbound first binder remains inward in the radial direction of the reaction chamber 70. Here, the amount of the bound first binder that forms the first composite and precipitates is directly proportional to an amount of a target material contained in the supernatant. Since a decrease in the amount of the first binder in the reaction chamber 70 causes a decrease in absorbance or an increase in amount of transmitted light, a concentration of the target material in the sample may be determined by measuring the foregoing levels and preparing a standard curve with the measured values.

In this regard, when the difference in specific gravities of the first composite and the first binder is significant, these materials may be separated from each other by centrifugation and a light absorbance or transmittance of a supernatant is determined as described above and in turn enables measurement of a concentration of the target material such as allergen. On the other hand, if the difference is not so great and successful separation is not attained, light amount measurement, turbidity measurement, fluorescence detection, etc. is used to calculate quantitative values of such materials.

More particularly, a conventional method such as light amount measurement, turbidity measurement, slide agglutination, etc. is well known and detects an agglutinated composite generated by the reaction of an antigen with an antibody. Since these methods are carried out after the antigen and the antibody are homogeneously dispersed in a solution, the foregoing techniques generally refer to a method for measurement of homogeneous immune reaction. According to such immune reaction, an agglutinated composite is produced and a reaction system causes turbidity based on amount of antigen and/or antibody. A turbidity method comprises optically measuring turbidity while the light amount measurement method is based on an amount of scattered light in the reaction system. More particularly, the turbidity method is a method for determining turbidity based on a decrease in the amount of transmitted light due to light scattering in the reaction system. Alternatively, the slide agglutination method comprises visibly monitoring turbidity caused by agglutinated composite on a glass slide.

Fluorescence detection is a method for measurement of fluorescent sensitivity using a light source such as a laser or fluorescent lamp (not shown), an optical filter (not shown), etc. by extracting fluorescent light from excited light.

Meanwhile, a test apparatus having a micro-fluidic device according to an exemplary embodiment of the present invention can detect light at a wavelength of 300 to 900nm.

According to another exemplary embodiment of the present invention, a dilution chamber 60 receives a first binder, which reacts with a target material contained in a supernatant through antigen-antibody reaction to form a first antigen-antibody complex, as well as a desired amount of a dilution buffer, in consideration of the dilution ratio of the supernatant required for assay.

Accordingly, the supernatant entered into the dilution chamber 60 reacts with the first binder in the same chamber to form a first composite.

At least two reaction chambers 70 are located radially outward the dilution chamber 60. The reaction chambers 70 are connected to the dilution chamber 60 via a dispenser channel 61. Distribution of a diluted sample through the dispenser channel 61 may be controlled by a valve 62. An alternative valve 63 serves to provide an air vent path to easily dispense the diluted sample into the reaction chambers 70. Each of such valves 62 and 63 may be a micro-fluidic valve with the same morphology as that of the valve 35.

The reaction chambers 70 contains a first binder and a second binder as reagents in a liquid or dried solid state wherein the first binder and the second binder bind to different sites of the target material.

The second binder is coupled to a particle having relatively higher specific gravity, for example, iron oxide/polystyrene nano-shell, than a particle of the first binder.

The reagent containing the second binder may be introduced into the reaction chamber before an upper plate is combined with a lower plate to form a platform 100 during manufacture of a micro-fluidic device to produce a micro-fluid device preloaded with the reagent. Alternatively, the reaction chamber 70 may be a chamber having a vent and an inlet instead of a closed reaction chamber and, in this case, the reagent may be introduced into the reaction chamber 70 through the vent or inlet before being subjected to assay.

For instance, before both upper and lower plates are combined form a platform 100 during manufacture of a micro-fluidic device, a liquid reagent containing a second binder is introduced into at least two reaction chambers 70 and those contained in the reaction chambers are simultaneously subjected to lyophilization according to a known lyophilizing program. Afterward, binding the upper and lower plates together, a micro-fluidic device 200 containing the lyophilized reagent therein may be fabricated.

The lyophilized reagent may be prepared by adding a filler and a surfactant to a liquid reagent and then lyophilizing the mixture. The filler assists the formation of a porous structure of the lyophilized reagent, so as to facilitate further dissolution of a mixture containing a sample and a dilution buffer or a blend of this mixture and a first composite when these materials were introduced into the reaction chamber 70. The filler may be selected from: bovin serum albumin (BSA); polyethylene glycol (PEG); mannitol; polyalchol; myo-inositol; citric acid; ethylenediamine tetraacetic acid disodium salt (EDTA2Na); polyoxyethyleneglycol dodecylether (BRIJ-35); sucrose; and trehalose. Such filler may be added alone or in combination of two or more thereof depending on type of the reagent. Also, the surfactant may be selected from: polyoxyethylene; laurylether; octooxynol; polyethylene alkylalcohol; nonylphenol polyethyleneglycol ether; ethylene oxide; ethoxylated tridecylalcohol; polyoxyethylene nonylphenylether phosphate sodium salt; and sodium dodecyl sulfate. Such surfactant may be added alone or in combination of two or more thereof depending on type of the reagent.

After a target material in a sample is combined with the first binder to form the first composite, a valve 62 is opened and the first composite flows toward the reaction chambers 70 and in turn dissolves the second binder in a dried state, thus forming a second composite.

For the above purpose, the first composite and the second binder may be allowed to be contact each other for a desired period of time, so as to form the second composite.

Next, after the formation of the second composite, the micro-fluidic device 200 rotates to enable centrifugation of the composite.

According to rotation of the micro-fluidic device 200, the second composite moves outwardly in a radial direction of the reaction chamber 70 because of its higher gravity while the unbound first binder which does not form the second composite remains inward in the radial direction of the reaction chamber 70. Here, the amount of the second binder that forms the second composite and precipitates is directly proportional to the amount of the target material contained in the supernatant. Since a decrease in the amount of the first binder causes a decrease in the absorbance or an increase in the amount of transmitted light, the concentration of the target material in a specimen may be determined by measuring the foregoing levels and providing a standard curve prepared from measured values of the target material of previously known concentrations.

In this regard, when the difference in specific gravities of first solid particle coupled to the first binder and second solid particle coupled to the second binder is significant, these materials may be separated by centrifugation and light absorbance of a supernatant is determined as described above, in turn enabling measurement of a concentration of, for example, allergen. On the other hand, if the difference is not so great and separation is not attained, light amount measurement, turbidity measurement, fluorescence detection, etc. is used to calculate quantitative values of such materials.

According to the exemplary embodiment, the first binder and the second binder may be prepared by the following procedures:

### 1. Preparation of first binder

10ml of a solution containing colloidal gold particles (with specific gravity in a range which prevent the particles from sinking in the normal gravity) having an average particle diameter of 20nm (British Biocell, P.N., GC 20) was mixed with 25*µ*g of purified monoclonal anti-Human IgE (Medix Biocemica, P.N. 100104) and the mixture was agitated at room temperature for 30 minutes, so as to enable adsorption of the above antibody to a surface of the gold particle. After removing excess antibody not coupled to the surface of the particle, the first binder suspended at a concentration of OD520=10 was prepared.

### 2. Preparation of second binder

900*µ*ℓ of iron oxide particles having an average particle diameter of 1,000nm and a solid density of 1.8mg/cm³, (Dynal Biotech, P.N. 650.11) was mixed with 25mM of MES saline and a buffer (pH 4.5) and the mixture was washed three times. After suspending the particles at 1%(w/v) concentration, 10mg of EDC(1-ethyl-3-[3-dimethylaminopropryl]carbodiimide hydrochloride) and 10mg of NHC(N-hydroxysuccinimide) were added and agitated at 37°C for 30 minutes so as to activate a surface of the particle. After washing the surface activated particles three times, the washed particles were divided in equal amounts and entered into three test tubes. Purified allergy antigens *Der p, Der f* and *Alt* a were added with each amount of 150*µ*g to the above prepared three test tubes, respectively, followed by agitation of the mixture in each test tube at room temperature for 30 minutes to allow, thus allowing the allergy antigens to bind to the surface of the particle. After washing and removing excess allergy antigen not coupled to the surface of the particle, the second binder suspended at a concentration of 10nm/ml was prepared.

An illustrative example of immunoassay may be conducted as described below.

### 3. Immunoassay procedure

After 10*µ*g of each of the second binders prepared above is mixed with a desired filler and injected into each of separate reaction chambers 70, the mixture contained in the chamber is subjected to lyophilization. 0.89ml of dilution buffer (1% BSA, 0.5% Tween 20 in PBS) is mixed with 0.1ml of the prepared first binder and the mixture is injected into a dilution chamber 60. 0.43ml of whole blood containing an anticoagulant is added through an inlet 11 into a sample chamber 10 and is transported to a sample dispenser 30. Rotating a micro-fluidic device 200, the blood is subjected to centrifugation and in turn is separated into blood cell components and a supernatant. After collecting the supernatant in a supernatant collector 31 and measuring its amount in a metering chamber 50, the metered supernatant sample is transported into the dilution chamber 60 in which the sample is incubated for a predetermined period of time to allow reaction of a target material in the sample, that is, human IgE with the first binder. At least one chamber among the reaction chambers 70 is empty and, after completion of the reaction, is used for measuring a reference absorbance of a first composite. After the target material (IgE) in the sample binds to the first binder to form a first composite (i.e., a complex of IgE and anit-IgE-antibody coupled to gold particle) and a valve 62 is open, the first composite flows into the reaction chambers 70 and in turn dissolves a solid second binder, thus forming the second composite.

For this purpose, the first composite and the second binder in the reaction chamber 70 are allowed to be in contact each other for a desired period of time, so as to form a second composite.

Next, after formation of the second composite, the micro-fluidic device 200 rotates to separate the second composite from the unbound second binder. For each of the reaction chambers 70 as well as a reference chamber, absorbance is measured. Then, a difference in absorbance between the reference chamber and each of the reaction chambers 70 containing the second binder is determined and the determined value is substituted into a pre-prepared calibration curve to calculate a quantitative level of allergen (D*er p, Der f, Alt a*) specific Human IgE. Results of preparing a calibration curve are shown in TABLE 2.

### Table 2

### [Table 2]

**[Table]**

| Human IgE (ng/ml) | A540 | Blank - A540 |
|---|---|---|
| 100 | 0.239 | 0.043 |
| 50 | 0.259 | 0.022 |
| 25 | 0.262 | 0.019 |
| 12.5 | 0.276 | 0.005 |
| 0 | 0.281 | 0.000 |

A reference unit 103 (FIG. 2) containing no sample may be placed on the platform 100. A dilution chamber 80 may contain a dilution buffer stored to obtain a standard value during detection of reaction. Individual chambers 90 which are empty or filled with the dilution buffer may be arranged radially outward the dilution chamber 80 to determine the standard value. The chambers 90 are connected to the dilution chamber 80 via a channel 81. The channel 81 may have a valve 82. An alternative valve 83 serves to form an air vent path. Each of such valves 82 and 83 may be identical to the valve 35. A chamber 91 positioned at an end of the channel 81 serves to enable monitoring of whether the dilution buffer is fully charged in the chambers 90.

Although not described in detail, the micro-fluidic device may have air vent paths for discharging air charged therein.

FIG. 6 is a block diagram illustrating a test system using a micro-fluidic device according to an exemplary embodiment.

A blood test system according to the exemplary embodiment of may include a rotational driver 301 to rotate a disk type micro-fluidic device, a valve switching device 310 to open and close valves, an inspection part 320, an output part 330, a diagnosis DB 340 and a control part 350 for controlling such constructional components.

The rotational driver 301 rotates the disk type micro-fluidic device to provide centrifugal force required for centrifugation of the sample and movement of a fluid. The rotational driver 301 also stops or rotates the disk type micro-fluidic device to locate or align the reaction chamber 70 to a desired position.

Although not shown, the rotational driver 301 may have a motor driving device to control angular position of the disk type micro-fluidic device 10. For instance, the motor driving device may be a stepper motor or a DC motor.

The valve switching device 310 opens or closes valves 35, 51 and 62 of the disk type micro-fluidic device 200 and may include an external energy source 303 and a moving unit 304 to transport the external energy source 303 to one of the valves to be open.

The external energy source 303 may be a laser source to radiate a laser beam, a light emitting diode to radiate visible or infrared light, a xenon lamp, and the like. In particular, the laser source may have at least one laser diode.

The moving unit 304 may include a driving motor (not shown) and a gear part (not shown) equipped with the external energy source 303 to move the external energy source 303 above one of the valves to be opened by rotation of the driving motor.

The inspection part 320 may be installed in plural to determine absorbance of the reaction chamber and at least one light emission part 321, which corresponds to the light emission part 321, and at least one light receiving part 322, which receives light penetrating the reaction chamber 70 of the micro-fluidic device 200.

The light emission part 321 may be a light source flashing at a specific frequency including, for example, a semiconductor light emitting device such as an LED or a laser diode (LD), a gas discharge lamp such as a halogen lamp or a xenon lamp, etc.

The light emission part 321 is placed on a site at which light emitted from the light emission part 321 passes through the reaction chamber 70 and reaches the light receiving part 322.

The light receiving part 322 generates electrical signals according to an intensity of incident light and adopts, for example, a depletion layer photodiode, avalanche photodiode (APD), photomultiplier tube (PMT), etc.

In the present exemplary embodiment, the light emission part 321 is located above the disk type micro-fluidic device 200 while the light receiving part 322 is positioned below the disk type micro-fluidic device 200, however, the positions of these parts may be switched. Also, a light path may be adjusted using a reflecting mirror or a light guide member (not shown).

The control part 350 controls the rotational driver 301, the valve switching device 310 and/or the inspection part 320 to smoothly conduct operation of the test system, searches the diagnostic DB 340 and uses absorbance detected from the inspection part 320 and a standard curve stored in the diagnostic DB 340 so as to determine the concentration of a target material in the supernatant contained in the reaction chamber 70 of the micro-fluidic device 200.

The output part 330 outputs diagnosed results and information as to whether the diagnosis is completed or not and may comprise a visible output device such a liquid crystal display (LCD), an audio output device such as a speaker, or an audiovisual output device.

The following description will be given of an immunosorbent assay method using the foregoing micro-fluidic device. According to an exemplary embodiment, a process for blood analysis will be described in detail.

For example, whole blood sampled from a subject to be tested is introduced into the sample chamber 10 and the micro-fluidic device is installed on the rotational driver 301.

Next, rotating the micro-fluidic device 200 at a low speed, the blood sample is transported from the sample chamber 10 to the sample dispenser 30. The low speed means a revolution speed generating centrifugal force suitable for moving a fluid. For example, the micro-fluidic device 200 may rotate with an accelerated velocity of 1800 rpm/10 sec for 11 seconds. By centrifugal force, the sample moves from the sample chamber 10 to the sample dispenser 30. After completely filling the sample dispenser 30, the sample is transported into the sample dispenser 30a via the sample transport part 20.

Then, a centrifuging process is conducted. The rotational driver 301 rotates the micro-fluidic device 200 at a high speed. Such high speed means a revolution speed separating the blood into a serum or plasma as a supernatant and a precipitate (blood cells). For example, the micro-fluidic device 200 may rotate with an accelerated velocity of 3600 rpm/10 sec for about 160 seconds. As a result, relatively heavy blood cells are moved into the precipitate collector 32 while the supernatant remains in the supernatant collector 31.

Using a valve switching device 310, electromagnetic waves are radiated to the closed valve 35. Subsequently, the valve material is melted and the valve is opened, as shown in FIG. 4. The rotational driver 301 rotates the micro-fluidic device 200 to generate centrifugal force. Then, the supernatant moves from the supernatant collector 31 to the supernatant metering chamber 50 via the channel 34. Since the valve 51 at an outlet of the supernatant metering chamber 50 is closed, the supernatant fills the supernatant metering chamber 50. Accordingly, if an amount of the supernatant is sufficient, the supernatant is contained in the supernatant metering chamber 50 to an amount corresponding to a volume of the same chamber.

Next, the valve 51 is opened using the valve switching device 310 and the supernatant moves from the supernatant metering chamber 50 to the dilution chamber 60 by rotation of the micro-fluidic device 200. Here, the valve 63 is also opened to form an air vent path. The rotational driver 301 may shake the micro-fluidic device 200 several times in right and left directions in order to mix a dilution buffer containing the first binder with the supernatant. As a result, a diluted sample containing a first composite obtained by the reaction of the sample with the first binder is present in the dilution chamber 60.

Subsequently, the valve 62 is opened using the valve switching device 310 and the diluted sample is charged in the reaction chambers 70 and an inspection chamber 46 for monitoring the diluted sample via the dispenser channel 34 by rotation of the micro-fluidic device. Also, using the valve switching device 310, the valves 82 and 83 are opened. According to rotation of the micro-fluidic device 200, the dilution buffer in the dilution chamber 80 fills the chamber 90 via the channel 81.

The rotational driver 301 may shake the micro-fluidic device 200 several times in right and left directions in order to mix the diluted sample containing the first composite with the second binder. Then, absorbance of each of the reaction chambers 70 and, if necessary, each of the chambers 90 of a reference unit 103 is determined using the inspection part 320. For analysis of end points, the absorbance is repeatedly measured at constant intervals to determine an absorbance during a saturated reaction. Based on information on a relationship between absorbance and concentration, which is stored in the diagnostic DB 340, a concentration of each of substances to be analyzed is calculated.

According to the present exemplary embodiment, biochemical analysis may be conducted using a biochemical analysis unit equipped with the same micro-fluidic structure as that of the IMU. In another embodiment, the biochemical analysis may be performed even using the IMU.

However, when the biochemical analysis is carried out using the IMU, the dilution chamber 60 of the micro-fluidic device 200 is used and the reaction chambers 70 may receive appropriate reagents and diluents for biochemical analysis.

The following description will be given to explain use of the biochemical analysis unit (BCU) of the micro-fluidic device.

Since the BCU has substantially the same structure as of the IMU, detailed description of configurations of the micro-fluidic device will be omitted, instead, replaced by the foregoing description for IMU. Accordingly, only a process for biochemical analysis will be described below.

The BCU may execute a variety of blood test items. Since a dilution ratio of blood to diluent (that is, dilution buffer) is altered according to the blood test items, at least two dilution chambers 65 are used to conduct test items requiring different dilution ratios, so as to correspond to at least two dilution ratios.

More particularly, albumin (ALB), amylase (AMY), blood urea nitrogen (BUN), calcium Ca⁺⁺, total cholesterol (CHOL), chloride Cl⁻, creatine (CRE), glucose (GLU), gamma glutamyl transferase (GGT), high-density lipoprotein cholesterol (HDL), potassium (K⁺), lactate dehydrogenase (LD), sodium (Na⁺), total carbon dioxide (TCO2), total protein (TP), triglycerides (TRIG), uric acid (UA), and the like require a dilution ratio of serum : dilution buffer = 1:100.

Alternatively, alanine aminotransferase (ALT), alkaline phosphatase (ALP), aspartate aminotransferase (AST), creatine kinase (CK), direct bilirubin (D-BIL) and total bilirubin (T-BIL) require a dilution ratio of serum : dilution buffer = 1:20. Accordingly, the dilution chamber 65 of the present exemplary embodiment may be used to examine test items requiring a dilution ratio of serum to dilution buffer of 1:100 and, alternatively, another dilution chamber may be further provided to correspond to a dilution ratio of serum to dilution buffer of 1:20.

The reaction chambers 84 may receive individual reagents reacting with the sample to cause different reactions, respectively. Such reagents may be introduced into the reaction chambers before upper and lower plates are combined to form the platform 100 during manufacture of a micro-fluidic device. Each of the reaction chambers 84 may be a chamber having a vent and an inlet instead of a closed reaction chamber. In this case, the reagents may be introduced into the reaction chambers 84 before assay. Each of such reagents may be in a liquid or lyophilized solid state.

For instance, before an upper plate is combined with a lower plate to form a platform 100 during manufacture of a micro-fluidic device, a liquid reagent is loaded into at least two reaction chambers 84 and those contained in the reaction chambers are simultaneously subjected to lyophilization according to a known lyophilizing program. Afterward, binding the upper and lower plates together, a micro-fluidic device 200 containing the lyophilized reagent therein may be fabricated. The lyophilized reagent may be prepared by adding a filler and a surfactant to a liquid reagent, followed by lyophilizing the resulting mixture.

The following description will be given to explain a biochemical analysis method using the foregoing micro-fluidic device. As an illustrative example, a process for analysis of blood will be described below.

The dilution chamber 65 of the micro-fluidic device may contain a dilution buffer beforehand. Otherwise, the dilution buffer may be introduced through an inlet (not shown) into the dilution chamber 60. Whole blood sampled from a subject to be tested is entered into the sample chamber 10 and the micro-fluidic device 200 is installed on the rotational driver 301.

Like the immunosorbent assay described above, rotating the micro-fluidic device 200 at a low speed, the blood sample is delivered from the sample chamber 10 to the sample dispenser 30. After completely filling the sample dispenser 30, the sample is transported into the sample dispenser 30a via the sample transport part 20. After completely filling the sample dispenser 30a, the blood moves through the channel 42 into an excess sample chamber 41.

Then, a centrifuging process is conducted by the same procedures for immunosorbent assay. The rotational driver 301 rotates the micro-fluidic device 200 at a high speed.

Using the valve switching device 310, electromagnetic waves are radiated to the closed valve 35a. Continuously, the valve material is fused and the valve 35a is opened, as shown in FIG. 5. The rotational driver 301 rotates the micro-fluidic device 200 to generate centrifugal force. Then, the supernatant moves from the supernatant collector 31 to the supernatant metering chamber 50a via the channel 34a. Since the valve 51a at an outlet of the supernatant metering chamber 50a is closed, the supernatant is charged in the supernatant metering chamber 50a.

Next, the valve 51a is opened using the valve switching device 310 and the supernatant moves from the supernatant metering chamber 50a to the dilution chamber 65 by rotation of the micro-fluidic device 200. Here, the valve 63a is also opened to form an air vent path. The rotational driver 301 may shake the micro-fluidic device 200 several times horizontally or in a seesaw movement in order to mix the dilution buffer with the supernatant. As a result, a diluted sample is present in the dilution chamber 65.

Subsequently, the valve 62a is opened using the valve switching device 310 and the diluted sample is filled in the reaction chambers 84 and an inspection chamber 46a for monitoring the diluted sample via the dispenser channel 61a by rotation of the micro-fluidic device. Also, using the valve switching device 310, the valves 82 and 83 are opened. According to rotation of the micro-fluidic device 200, the dilution buffer in the dilution chamber 80 fills the chamber 90 via the channel 81.

The rotational driver 301 may shake the micro-fluidic device 200 several times horizontally or in a seesaw movement in order to mix the diluted sample with a reagent. Then, absorbance of each of the reaction chambers 84 and each of the chambers 90 of the reference unit 103 is measured using the inspection part 320. Based on a relationship between absorbance and concentration stored in the diagnostic DB 340, a concentration of each of substances to be analyzed is calculated.

Although a few embodiments of the present invention have been shown and described in conjunction with accompanying drawings, these are proposed for illustrative purposes without restricting configurations or characteristics of the present invention and it would be appreciated by those skilled in the art that substitutions, variations and/or modifications may be made in these embodiments without departing from the scope of the invention as defined in the claims.

## Claims

1. A micro-fluidic device (200), comprising:
a rotational body (100);
a micro-fluidic structure placed on the rotational body, having multiple chambers (10, 41, 46a 50, 50a, 60, 65, 80, 84, 90), multiple channels (34, 34a, 42, 61, 61a, 81) through which a fluid flows between the multiple chambers, and multiple valves (35, 51, 62, 63, 82, 83) arranged in the channels to control a flow of the fluid, wherein the fluid is transported via centrifugal force generated by rotation of the rotational body; and
a first binder provided in a first chamber of the multiple chambers in the micro-fluidic structure wherein the binder comprises a first capture material that specifically binds to a first site of a target material to be detected and a first solid particle; and
a second binder provided in a second chamber of the multiple chambers in the micro-fluidic structure wherein the second binder is specifically binds to a second site of the target material to be detected, and wherein the second site of the target material is different from the first site of the target material;
**characterized in that**
the second binder is coupled to a second solid particle to which a second capture material binds, and the second solid particle has a specific gravity larger than that of the first binder.

2. The micro-fluidic device according to claim 1, wherein the first capture material is selected from the group consisting of an antibody, an antigen, an oligonucleotide and an aptamer.

3. The micro-fluidic device according to claim 1, wherein the first solid particle is a metal nanoparticle, a polymer particle or semiconductor nanocrystal.

4. The micro-fluidic device according to claim 3, wherein the first solid particle is a gold nano-particle.

5. The micro-fluidic device according to claim 1, wherein the second solid particle is a particle composed of an iron oxide as a core and a polymer shell covering the iron oxide core.

6. An immunosorbent assay method using a micro-fluidic device (200), comprising:
providing a micro-fluidic device (200) comprising:
a rotational body (100);
a micro-fluidic structure placed on the rotational body, having multiple chambers (10, 41, 46a 50, 50a, 60, 65, 80, 84, 90), multiple channels (34, 34a, 42, 61, 61a, 81) through which a fluid flows between the multiple chambers, and multiple valves (35, 51, 62, 63, 82, 83) arranged in the channels to control a flow of the fluid, wherein the fluid is transported via centrifugal force generated by rotation of the rotational body;
a first binder provided in a first chamber of the multiple chambers in the micro-fluidic structure wherein the binder comprises a first capture material that specifically binds to a first site of a target material to be detected and a first solid particle; and a second binder provided in a second chamber of the multiple chambers in the micro-fluidic structure wherein the second binder is specifically binds to a second site of the target material to be detected, and wherein the second site of the target material is different from the first site of the target material;
loading a fluid sample to the micro-fluidic device to bring the sample to be in contact with the first binder for a sufficient time to allow forming a first complex of the first binder and a target material contained in the sample; bring the first complex to be in contact with the second binder for a sufficient time to allow to form a second complex of the first complex and the second binder;
rotating the micro-fluidic device to separate the second complex as a precipitate from the sample, producing a supernatant and the precipitate; and
determining optical properties of supernatant and/or the precipitate;
**characterized in that**
the second binder is coupled to a second solid particle to which a second capture material binds and the second solid particle has a specific gravity larger than that of the first binder.

7. The method according to claims 6, wherein the first binder and the second binder are selected from the group consisting of an antibody, an antigen, an oligonucleotide and an aptamer.

8. The method according to claim 7, wherein the solid particle is selected from the group consisting of a metal nanoparticle, a polymer particle and a semiconductor nanocrystal.

9. The method according to claim 8, wherein the solid particle is a gold nanoparticle.

10. The method according to claim 6, wherein the second solid particle is a particle composed of an iron oxide as a core and a polymer shell covering the iron oxide core.

11. The method according to claim 6, wherein a fraction is present radially inside the micro-fluidic device within the chamber.

12. The method according to claim 6, further comprising: shaking the micro-fluidic device horizontally or a seesaw movement, while reactions are carried out in the first or second chamber.

13. The method according to claim 6, further comprising separating a supernatant from the sample, before bringing the sample to be contact with the first binder.

14. The method according to claim 6, wherein the micro-fluidic device further comprises an additional micro-fluidic structure comprising a reagent, said reagent binding to the target material in the sample, and wherein the method further comprising concurrently or sequentially loading the sample to the additional micro-fluidic structure to bring the reagent to be in contact with the sample.

## Patentansprüche

1. Mikrofluidische Vorrichtung (200), die umfasst:
einen Drehkörper (100);
eine auf dem Drehkörper angeordnete mikrofluidische Struktur aufweisend mehrere Kammern (10, 41, 46a, 50, 50a, 60, 65, 80, 84, 90), mehrere Kanäle (34, 34a, 42, 61, 61a, 81), durch die ein Fluid zwischen den mehreren Kammern strömt, und mehrere Ventile (35, 51, 62, 63, 82, 83), die in den Kanälen zum Steuern einer Strömung des Fluids ausgerichtet sind, wobei das Fluid mittels durch eine Drehbewegung des Drehkörpers erzeugte Zentrifugalkraft transportiert wird;
ein erstes Bindemittel, das in einer ersten Kammer aus der Menge der mehreren Kammern in der mikrofluidischen Struktur vorgesehen ist, wobei das Bindemittel ein erstes Einfangmaterial, welches sich speziell an eine erste Stelle eines zu erfassenden Zielmaterials bindet, und ein erstes festes Teilchen umfasst; und
ein zweites Bindemittel, das in einer zweiten Kammer aus der Menge der mehreren Kammern in der mikrofluidischen Struktur vorgesehen ist, wobei das zweite Bindemittel sich speziell an eine zweite Stelle des zu erfassenden Zielmaterials bindet, und wobei die zweite Stelle des Zielmaterials sich von der ersten Stelle des Zielmaterials unterscheidet;
**dadurch gekennzeichnet, dass**
das zweite Bindemittel mit einem zweiten festen Teilchen, an das sich ein zweites Einfangmaterial bindet, gekoppelt ist, und dass das zweite feste Teilchen eine relative Dichte aufweist, die größer als die des ersten Bindemittels ist.

2. Mikrofluidische Vorrichtung nach Anspruch 1, wobei das erste Einfangmaterial aus der Gruppe bestehend aus Antikörper, Antigen, Oligonukleotid und Aptamer ausgewählt wird.

3. Mikrofluidische Vorrichtung nach Anspruch 1, wobei es sich bei dem ersten festen Teilchen um ein Metall-Nanoteilchen, ein Polymerteilchen oder einen Halbleiter-Nanokristall handelt.

4. Mikrofluidische Vorrichtung nach Anspruch 3, wobei es sich bei dem ersten festen Teilchen um ein Gold-Nanoteilchen handelt.

5. Mikrofluidische Vorrichtung nach Anspruch 1, wobei es sich bei dem zweiten festen Teilchen um ein Teilchen handelt, das aus einem Kern aus Eisenoxid und einer Polymerumhüllung, die den Eisenoxidkern umgibt, besteht.

6. Immunosorbent-Assay-Verfahren unter Verwendung einer mikrofluidischen Vorrichtung (200), wobei das Verfahren umfasst:
Bereitstellen einer mikrofluidischen Vorrichtung (200), die umfasst:
einen Drehkörper (100);
eine auf dem Drehkörper angeordnete mikrofluidische Struktur aufweisend mehrere Kammern (10, 41, 46a, 50, 50a, 60, 65, 80, 84, 90), mehrere Kanäle (34, 34a, 42, 61, 61a, 81), durch die ein Fluid zwischen den mehreren Kammern strömt, und mehrere Ventile (35, 51, 62, 63, 82, 83), die in den Kanälen zum Steuern einer Strömung des Fluids ausgerichtet sind, wobei das Fluid mittels durch eine Drehbewegung des Drehkörpers erzeugte Zentrifugalkraft transportiert wird;
ein erstes Bindemittel, das in einer ersten Kammer aus der Menge der mehreren Kammern in der mikrofluidischen Struktur vorgesehen ist, wobei das Bindemittel ein erstes Einfangmaterial, welches sich speziell an eine erste Stelle eines zu erfassenden Zielmaterials bindet, und ein erstes festes Teilchen umfasst; und
ein zweites Bindemittel, das in einer zweiten Kammer aus der Menge der mehreren Kammern in der mikrofluidischen Struktur vorgesehen ist, wobei das zweite Bindemittel sich speziell an eine zweite Stelle des zu erfassenden Zielmaterials bindet, und wobei die zweite Stelle des Zielmaterials sich von der ersten Stelle des Zielmaterials unterscheidet;
Beladen der mikrofluidischen Vorrichtung mit einer Fluidprobe, so dass die Probe lange genug mit dem ersten Bindemittel in Kontakt gebracht wird, um die Bildung eines ersten Komplexes aus dem ersten Bindemittel und einem in der Probe enthaltenen Zielmaterial zu ermöglichen, und so dass der erste Komplex lange genug in Kontakt mit dem zweiten Bindemittel gebracht wird, um die Bildung eines zweiten Komplexes aus dem ersten Komplex und dem zweiten Bindemittel zu ermöglichen;
Drehen der mikrofluidischen Vorrichtung, um den zweiten Komplex als Ausscheidung von der Probe abzuscheiden, wodurch ein Überstand und die Ausscheidung erzeugt werden; und
Bestimmen optischer Eigenschaften des Überstands und/oder der Ausscheidung;
**dadurch gekennzeichnet, dass**
das zweite Bindemittel mit einem zweiten festen Teilchen, an das sich ein zweites Einfangmaterial bindet, gekoppelt ist, und dass das zweite feste Teilchen eine relative Dichte aufweist, die größer als die des ersten Bindemittels ist.

7. Verfahren nach Anspruch 6, wobei das erste Bindemittel und das zweite Einfangmaterial aus der Gruppe bestehend aus Antikörper, Antigen, Oligonukleotid und Aptamer ausgewählt werden.

8. Verfahren nach Anspruch 7, wobei das feste Teilchen aus der Gruppe bestehend aus einem Metall-Nanopartikel, einem Polymer-Nanopartikel und einem Halbleiter-Nanokristall ausgewählt wird.

9. Verfahren nach Anspruch 8, wobei es sich bei dem ersten festen Teilchen um ein Gold-Nanoteilchen handelt.

10. Verfahren nach Anspruch 6, wobei es sich bei dem zweiten festen Teilchen um ein Teilchen handelt, das aus einem Kern aus Eisenoxid und einer Polymerumhüllung, die den Eisenoxidkern umgibt, besteht.

11. Verfahren nach Anspruch 6, wobei eine Fraktion radial im Inneren der mikrofluidischen Vorrichtung innerhalb der Kammer vorhanden ist.

12. Verfahren nach Anspruch 6, das weiterhin umfasst: Schütteln der mikrofluidischen Vorrichtung horizontal oder mit einer Wippbewegung, während Reaktionen in der ersten oder zweiten Kammer stattfinden.

13. Verfahren nach Anspruch 6, das weiterhin umfasst: Abscheiden eines Überstandes aus der Probe, bevor die Probe mit dem ersten Bindemittel in Kontakt gebracht wird.

14. Verfahren nach Anspruch 6, wobei die mikrofluidische Vorrichtung weiterhin eine zusätzliche mikrofluidische Struktur umfassend ein Reagens umfasst, wobei das Reagens sich an das Zielmaterial in der Probe bindet, und wobei das Verfahren weiterhin umfasst: gleichzeitiges oder aufeinander folgendes Beladen der zusätzlichen mikrofluidischen Struktur mit der Probe, um das Reagens mit der Probe in Kontakt zu bringen.

## Revendications

1. Dispositif microfluidique (200), comprenant :
un corps rotatif (100),
une structure microfluidique placée sur le corps rotatif, présentant de multiples chambres (10, 41, 46a, 50, 50a, 60, 65, 80, 84, 90), de multiples canaux (34, 34a, 42, 61, 61a, 81) dans lesquels un fluide s'écoule entre les multiples chambres, et de multiples vannes (35, 51, 62, 63, 82, 83) agencées dans les canaux pour réguler l'écoulement du fluide, ledit fluide étant déplacé sous l'effet de la force centrifuge produite par la rotation du corps rotatif,
un premier liant présent dans une première chambre des multiples chambres de la structure microfluidique, le liant comprenant un premier matériau de captage, qui se lie spécifiquement à un premier site d'un matériau cible à détecter, et une première particule solide, et
un deuxième liant présent dans une deuxième chambre des multiples chambres de la structure microfluidique, le deuxième liant se liant spécifiquement à un deuxième site du matériau cible à détecter, le deuxième site du matériau cible étant différent du premier site du matériau cible ;
**caractérisé en ce que**
le deuxième liant est couplé à une deuxième particule solide à laquelle se lie un deuxième matériau de captage, et la deuxième particule solide présente une densité relative supérieure à celle du premier liant.

2. Dispositif microfluidique selon la revendication 1, dans lequel le premier matériau de captage est choisi dans le groupe constitué par un anticorps, un antigène, un oligonucléotide et un aptamère.

3. Dispositif microfluidique selon la revendication 1, dans lequel la première particule solide est une nanoparticule de métal, une particule de polymère ou un nanocristal semiconducteur.

4. Dispositif microfluidique selon la revendication 3, dans lequel la première particule solide est une nanoparticule d'or.

5. Dispositif microfluidique selon la revendication 1, dans lequel la deuxième particule solide est une particule composée d'un noyau d'oxyde de fer et d'une enveloppe polymère recouvrant le noyau d'oxyde de fer.

6. Procédé de dosage immunoadsorbant au moyen d'un dispositif microfluidique (200), comprenant :
la mise en place d'un dispositif microfluidique (200) comprenant :
un corps rotatif (100),
une structure microfluidique placée sur le corps rotatif, présentant de multiples chambres (10, 41, 46a, 50, 50a, 60, 65, 80, 84, 90), de multiples canaux (34, 34a, 42, 61, 61a, 81) dans lesquels un fluide s'écoule entre les multiples chambres, et de multiples vannes (35, 51, 62, 63, 82, 83) agencées dans les canaux pour réguler l'écoulement du fluide, ledit fluide étant déplacé sous l'effet de la force centrifuge produite par la rotation du corps rotatif,
un premier liant présent dans une première chambre des multiples chambres de la structure microfluidique, le liant comprenant un premier matériau de captage, qui se lie spécifiquement à un premier site d'un matériau cible à détecter, et une première particule solide, et
un deuxième liant présent dans une deuxième chambre des multiples chambres de la structure microfluidique, le deuxième liant se liant spécifiquement à un deuxième site du matériau cible à détecter, le deuxième site du matériau cible étant différent du premier site du matériau cible ;
le chargement d'un échantillon de fluide dans le dispositif microfluidique afin de mettre en contact l'échantillon avec le premier liant pendant une durée suffisante pour former un premier complexe entre le premier liant et un matériau cible renfermé par l'échantillon, et de mettre en contact le premier complexe avec le deuxième liant pendant une durée suffisante pour former un deuxième complexe entre le premier complexe et le deuxième liant ;
la mise en rotation du dispositif microfluidique afin de séparer le deuxième complexe de l'échantillon sous la forme d'un précipité, en produisant un surnageant et ledit précipité ; et
la détermination de propriétés optiques du surnageant et/ou du précipité ;
**caractérisé en ce que**
le deuxième liant est couplé à une deuxième particule solide à laquelle se lie un deuxième matériau de captage, et la deuxième particule solide présente une densité relative supérieure à celle du premier liant.

7. Procédé selon la revendication 6, dans lequel le premier liant et le deuxième liant sont choisis dans le groupe constitué par un anticorps, un antigène, un oligonucléotide et un aptamère.

8. Procédé selon la revendication 7, dans lequel la particule solide est choisie dans le groupe constitué par une nanoparticule de métal, une particule de polymère et un nanocristal semiconducteur.

9. Procédé selon la revendication 8, dans lequel la particule solide est une nanoparticule d'or.

10. Procédé selon la revendication 6, dans lequel la deuxième particule solide est une particule composée d'un noyau d'oxyde de fer et d'une enveloppe polymère recouvrant le noyau d'oxyde de fer.

11. Procédé selon la revendication 6, dans lequel une fraction est présente radialement à l'intérieur du dispositif microfluidique au sein de la chambre.

12. Procédé selon la revendication 6, comprenant en outre : l'agitation du dispositif microfluidique à l'horizontale ou selon un mouvement de bascule, tandis que les réactions se produisent dans la première ou la deuxième chambre.

13. Procédé selon la revendication 6, comprenant en outre la séparation d'un surnageant de l'échantillon, avant de mettre l'échantillon en contact avec le premier liant.

14. Procédé selon la revendication 6, dans lequel le dispositif microfluidique comprend en outre une structure microfluidique supplémentaire, comprenant un réactif, ledit réactif se liant au matériau cible présent dans l'échantillon, et dans lequel le procédé comprend en outre le chargement, de façon concomitante ou successive, de l'échantillon dans la structure microfluidique supplémentaire afin de mettre le réactif en contact avec l'échantillon.
